# EUROPEAN PATENT APPLICATION

(11) **EP 1 170 378 A1**
(43) Date of publication of application: **09.01.2002**
(21) Application number: 00113462.6
(22) Date of filing: 26.06.2000
(51) Int. Cl.: C12Q 1/68

(54) **Method for monitoring the effect of cancer therapies**

(71) Applicant: Boehringer Ingelheim International GmbH, 55218 Ingelheim am Rhein (DE)
(72) Inventor: The designation of the inventor has not yet been filed
(74) Representative: Laudien, Dieter, Dr.

(57) **Abstract**

A method for monitoring the effect of cancer therapies with growth factor inhibitors and intracellular signal transduction modulators (,,growth factor cancer drugs"), which act by interfering with overexpression of normal or mutated forms of growth factor receptors, the overproduction of growth factors or defects in the signalling cascade downstream the activated growth factor receptors. The method is based on measuring telomerase activity in a sample from an individual diagnosed for cancer and treated with the growth factor cancer drug and correlating a reduction of telomerase activity with a favourable therapeutic intervention with the drug.

## Description

The present invention generally relates to the field of cancer therapy. In particular, the present invention relates to a method for monitoring the effect of certain cancer therapies.

Proliferation and differentiation of normal mammalian cells is a complex process regulated by the co-ordinated expression of a number of genes, including those coding for growth factors and growth factor inhibitors, and their corresponding receptors.

There is increasing evidence from studies in cancer biology that the deregulation of the co-ordinated expression of these genes, e.g. amplification, translocation, deletion or silencing of one or more genes and/or by their increased transcription, plays an important role in the development of certain types of cancer. For example, the observation that certain transformed or malignant cell lines require less serum (i.e. exogenous growth factors) for optimal growth in culture than their normal counterparts, led to the conclusion that human tumour cells have the ability to determine their destiny by producing their own growth factors.

It has also been observed in a number of cancers that a proto-oncogene is amplified and converted into an oncogene, which leads to the overexpression and/or mutated form of a growth factor or its receptor. Furthermore, changes in intracellular signal transduction pathways downstream the activated growth factor receptors, such as the ras pathways or the MAP kinase pathway, have been implicated in tumor development.

All these observations have led to the generation of a new type of cancer drugs, e.g. growth factor inhibitors and intracellular signal transduction modulators, which act by interfering with the consequences of the above-mentioned deregulation of genes, i.e. overexpression of normal or mutated forms of growth factor receptors, the overproduction of growth factors, defects in the signalling cascade downstream the activated growth factor receptor.

Growth factors are members of multifunctional polypeptide families that are involved in a variety of physiological processes including embryogenesis, cell proliferation and differentiation, angiogenesis and activation of the host immune system.

Based upon their structure and biological activity, the different growth factors have been grouped into separate families. These comprise the epidermal growth factor (EGF) family, the fibroblast growth factor (FGF) family, the platelet-derived growth factor (PDGF) family, the insulin-like growth factor (IGF) family, the neurotrophic factors (NGF) family, the transforming growth factor β (TGFβ) family and the haematopoietic growth factors.

Upon binding of a growth factor to its respective receptor, the receptor transmits a signal from its extracellular domain across the plasma membrane to the cytoplasmic domain, to which a tyrosine kinase activity is associated. Activation of the tyrosine kinase triggers a number of enzymatic reactions (via the Ras pathways or MAP kinase pathways) and biological effects, ultimately leading to DNA synthesis and cell proliferation.

Due to these characteristics, overexpressed growth factor receptors with tyrosin kinase activity have been recognized as promising targets for tumor therapy (Pharmacol.Ther.82:241, 1999; Pharmacol Ther. 82, 231, 1999;Curr.Opin.Oncol. 9,562,1997).

Consequently, chemical compounds that inhibit the tyrosine kinase activity of the growth factor receptors, antibodies directed to the extracellular domain of a growth factor receptor or the growth factors (or active fragments thereof) themselves have been suggested for the therapy of aberrant proliferation (Pharmacol.Ther.82,241,1999). In addition, compounds that interfere with components of the deregulated intracellular signal transduction pathway downstream the receptor tyrosine kinase activity, e.g. inhibitors of components of the Ras-pathways (i.e. famesyl transferase inhibitors) or of the MAP kinase pathways (i.e. MEK-kinase inhibitor) are suitable for therapeutic treatment of malignancies. In the following, the term "growth factor cancer drugs" is used as a synonym for compounds that act by a mechanism defined above.

A major problem in many anticancer treatments is the general unpredictability of their therapeutic effect. The therapeutic effects of traditional chemotherapeutical or radiation treatment are typically assessed by their effects on tumour size, i.e. the objective response. Hard data for therapeutic effectiveness are obtained by analyses of survival of treated patients. However, survival studies implicate large patient groups and long observation times. Therefore, there have been made attempts to find markers for the efficacy of cancer treatment (J Natl Cancer Inst 92, 205,2000).

A typical feature of the vast majority of human tumours and immortalized cells is the presence of active telomerase (PNAS 992, 9082, 1995; J. Natl Cancer Inst. 87,895,1995; Ann Med 30, 419, 1998). Telomerase is a ribonucleoprotein enzyme which utilizes its own RNA as a template to add hexanucleotide to the ends of replicating chromosomes compensating for the loss of telomeric DNA after each cell division (Nat. Med. 1,249,1995; Cancer Res. 55,2734, 1995; Annu. Rev. Biochem. 61,113, 1992). Telomerase maintains telomere length and chromosome stability, which are required for cellular immortalisation and subsequent malignant transformation. Thus, telomerase activity has been detected in the vast majority of human tumours. Telomerase is not expressed by normal postnatal human somatic cells, although low levels of telomerase activity can be detected in certain stem cells and activated cells of the haematopoietic system. Based on these observations, detection of telomerase activity has been suggested as a diagnostic marker for the presence of immortal cells, including certain types of cancer cells, present in normal tissue (Science 266,2011,1994).

There are several therapeutical interventions which have been shown to have an effect on telomerase activity. Firstly, drug-induced killing of tumor cells by chemotherapeutical agents, e.g. doxorubicin, cisplatin, has been shown to be associated with a decline in detectable telomerase activity. The decrease of telomerase levels paralleled cell growth impairment. The telomerase activity that was observed after treatment with chemotherapeutic agents most likely reflects the activity from the remaining viable cells. When tumor cells resistant to chemotherapeutic agents were treated with these compounds, no decline of telomerase activity or cell growth was observed. Similarly, high activity of telomerase in tumor tissue treated with 20 Gy of radiotherapy predicted a poor therapeutic effect of radiation and unfavourable patients' outcome for advanced cancers of oral cavity and oropharynx (Int J Molec Med 2, 301, 1998). Thus, these findings have provided a rationale for defining the level of telomerase activity during and after chemotherapy or radiation treatment as a marker for the success or failure of these therapies (Clin. Canc. Res. 3, 579, 1997; Int J Oncol 13, 489, 1998; Jpn J Cancer Res 89,1074,1998; Int J Cancer 79, 8, 1998).

Furthermore, it has been shown that the induction of senescence or the differentiation of immortal cells represses telomerase activity (PNAS 92, 12343, 1995; Leukemia 10, 1354, 1996). For instance, exposure of human acute promyelocytic leukemic *(NB-4)* and human embryonal carcinoma *(NTERA-2)* cells to the differentiation-inducers all-trans-retinoic acid or hexamethylene bisacetamide caused a decline in telomerase activity in differentiation-sensitive, but not in differentiation-resistant clones of these cells (Canc. Res. 56, 1503, 1996). These findings suggest the utility of measuring telomerase activity in leukemic cells for following disease progression and prediction of early cancer relapse (Clin. Canc. Res. 2, 799, 1996).

It has also been suggested that hormonal agents may directly or indirectly influence telomerase activity on certain tumor cell lines. The growth hormone-releasing hormone (GH-RH) antagonist MZ-5-156 was described to reduce telomerase activity of U-87 MG glioblastomas cultured in vitro (PNAS 96,226,1999). However, the pathway on which these substances exert their effect remains to be identified. The anti-estrogen tamoxifen was also shown to have an influence on telomerase activity in tumor cells. However, while a concentration of only 10⁻⁸M tamoxifen caused a repressive effect on telomerase activity in breast carcinoma cell lines MCF-7 and MDA-MB231, higher (10⁻⁷ and 10⁻⁶M) or lower (10⁻⁹M) concentrations of tamoxifen had no effect on telomerase activity (Cancer 85, 1523, 1999). The mechanism via which tamoxifen at 10⁻⁸ M affects telomerase activity also remains to be determined. Consequently, hormonal agents have been shown to influence telomerase activity in certain tumour cells via unknown mechanisms.

As opposed to classical chemotherapeutics or radiation, an objective response (defined as regression of tumor volume or eradication of cancer cells) during or after cancer therapy with growth factor cancer drugs cannot be seen in patients within a short period of time because these drugs have no immediate cell killing activity. For these drugs, it is expected that an objective response may be observed only after long term treatment. In the case the therapeutic effect due to the growth inhibitory properties of the compounds is a delay in tumour growth which results in prolonged time to progression or stable disease, it is even more difficult to rely on an objective parameter (J. Natl. Cancer Inst. 91, 1281, 1999). Furthermore, while telomerase activity has been suggested as a marker for monitoring chemotherapeutical and/or radiation treatments, in which the level of telomerase appears to be proportional to the number of the remaining tumor cells, no markers have been available for monitoring the therapeutic effect of growth factor cancer drugs.

It was an object of the present invention to provide a method of monitoring the effect of therapeutic intervention achieved by administration of growth factor cancer drugs.

The solution to the problem underlying the present invention is based on the unexpected finding that the inhibition of signaling triggered by growth factors such as EGF by growth factor cancer drugs not only reduces tumor cell proliferation, but also influences telomerase activity. The observed effect was highly surprising, because, as opposed to chemotherapeutics and radiation, growth factor cancer drugs do not act by simply killing the tumor cells, but by effecting various signalling pathways with no obvious link to the mechanism responsible for maintaining telomere length and chromosome stability.

It has been found in the present invention that the tumor cell growth modulating effects of growth factor cancer drugs can be monitored by determining the change in telomerase activity in cancer tissues/cells evoked by these drugs. Thus, telomerase activity can be utilized as a so-called "surrogate marker" for antitumor efficacy of these drugs.

The present invention relates to a method for monitoring and evaluating the efficacy of a growth factor cancer drug in therapy, said method comprising the steps of
a) collecting and preparing a sample containing cancer cells from an individual diagnosed for cancer and treated with a growth factor cancer drug,
b) determining the level of telomerase activity in said sample,
c) comparing the level of telomerase activity of said sample with the level determined in a sample in said individual before treatment or with a standard level of telomerase activity, and
d) correlating the level telomerase activity with the therapeutic effect of the growth factor cancer drug.

The method of the invention can be applied for monitoring the efficacy of the following growth factor cancer drugs : all growth factor cancer drugs which interfere with tyrosine kinase activities, and with components of the deregulated intracellular signal transduction pathways downstream the tyrosine kinase activities and with receptors related to the epidermal growth factor receptor (EGF-R) family, the fibroblast growth factor receptor (FGF-R) family, the platelet-derived growth factor receptor (PDGF-R) family, the insulin-like growth factor receptor (IGF-R) family, the neurotrophic factors receptor (NGF, BDNF,NT-3,4,5,6) family, the transforming growth factor β receptor (TGFβ) family and the haematopoietic growth factor receptors . Members of these receptor families are, for example: ErbB-1 (EGFR), ErbB-2 (neu/HER2), Erb-3 (HER3), Erb-4 (HER4), PDGFα and PDGFβ, FGF1, FGF2, FGF3, FGF4, TGFα and TGFβ, IGFR1, IGFR2, cytokine receptors (interferons, TNFalpha, interleukins) , trkA, trkB and trkC (receptors für neurotrophic factors ). In addition, the method of the invention may be applied to substances which interfere with the natural ligands binding to growth factor receptors mentioned above, i.e. inhibitors of the growth factors, like EGF, IGF or PDGF.

Examples for growth factor cancer drugs are growth factor receptor binding antibodies, e.g. antibodies against HER2, e.g. trastuzumab (Herceptin).

Further examples are chemical compounds known as inhibitors of the tyrosine kinase activities associated with the tyrosine kinase receptors (Expert Opinion On Investigational Drugs 1998, 7(4), 553 -573). Examples of tyrosine kinase inhibitors are also described in WO 96/07657, WO 97/01047, WO 97/01058, WO 97/01057, WO 96/33980, e.g. N-(3-chloro-4-fluorophenyl)-7-methoxy-6-[3-(4-morpholinyl)propoxy]-4-chinazolinamine (ZD-1839); WO 96/30347, e.g. N-(3-ethinylphenyl)-6,7-bis(2-methoxyethoxy)-4-chinazolinamine (CP 358774); WO 97/38983, e.g. N-(4-(3-(chloro-4-fluoro-phenylamino-7-(3-morpholine-4-yl-propoxy)-chinazoline-6-yl)-acrylamiddihydrochloride (CI 1033, PD 183805); WO 97/02266 (phenylaminopyrrolopyrimidine; PKI-166); examples of protein kinase inhibitors are also given in. Current Opinion in Drug Discovery & Development 1998 1 (2): 131-146

Examples for drugs that interfere with components of the deregulated intracellular signal transduction pathways downstream the tyrosine kinase activities are farnesyltransferase inhibitors, exemplified in PNAS 1994, 91(19), 9141-9145, or MEK inhibitors like the one described in J. Biol. Chem. 1995, 270 (46), 27489-27494.

In step a), the sample containing cancer cells is obtained by collecting cells from tissues obtained at surgery, or from biopsies, or collecting cells from body fluids (e.g. pleural, urine, blood) or washing solutions (e.g. lung, intraperitoneum or bladder). Cells can be collected by conventional methods, e.g. by centrifugation or other cell separation techniques, like FACS or magnetic beads, with or without the use of specific antibodies. The tissue samples are prepared in the usual manner: material extracts can be prepared by mincing, lysing with an appropriate buffer solution and homogenizing, or pulverizing after adding liquid nitrogen and resuspending in a lysis buffer, or lysing in a lysing buffer solution and homogenizing with a mechanical homogenizer. Another suitable method for sample preparation is the detergent-based extraction method, as described in WO 95/13381.

For determining the telomerase activity in step b) there are no limitations on the type of assay. It can be done according to any method known in the art or yet to be developed that is suitable for measuring telomerase activity.

A commonly used method, which is preferred in the present invention, is the telomerase extension assay, as described in Science 266, 2011, 1994, and in WO 95/13381, which is herein incorporated by reference. This method, which is commonly known as Telomeric Repeat Amplification Protocol (TRAP), is commercially available in the form of kits. It involves the extension of a nucleic acid substrate by telomerase and replication of extended substrates in a primer extension reaction, such as the polymerase chain reaction (PCR). This method provides a variety of means to quantitate the amount of telomerase in a sample. In a commercially available assay (TRAPEZE™ ELISA Telomerase detection kit, Oncor Inc.), in the first step telomerase adds a number of telomeric repeats (GGTTAG) onto the 3'end of a biotinylated telomerase substrate oligonucleotide. In the second step, the extended products are amplified by the polymerase chain reaction (PCR) using Taq polymerase, the telomerase substrate oligonucleotide, and reverse primers, and a nucleotide mix containing dCTP labeled with dinitrophenyl (DNP) residues. Thus, the labeled products are immobilized onto streptavidin-coated microtiter plates via biotin-streptavidin interaction and detected by an anti-DNP antibody conjugated to horseradish peroxidase. The amount of TRAP products is determined by means of the horseradish activity using substrate 3,3',5,5'-tetramethylbenzidine and subsequent colour development.

Alternatively, a direct visualisation of the TRAP ladder by polyacrylamid gel electrophoresis and staining with appropriate reagents (e.g. SYBR® Green or ethidium bromide) can easily be accomplished.

Another method for detecting telomerase activity that is useful in the present invention is described in WO 98/37241. This method comprises the steps of contacting a substrate nucleic acid, preferably a hairpin, with the sample and measuring the elongation of the nucleic acid, usually without employing replication steps like PCR.

Other methods for assaying telomerase activity in cell samples useful in the present invention rely on the incorporation of radioactively labelled nucleotides into a telomerase substrate (Cell 59,521,1989). The conventional assay uses an oligonucleotide substrate, a radioactive deoxyribonucleoside triphosphate (dNTP) for labeling, and gel electrophoresis. Although telomeric sequence oligonucleotides are efficient in vitro substrates, telomerase will also synthesize repeats using substrates comprosing non-telomeric DNA sequences.

Usually, according to step c), the telomerase activity in a sample can be measured before and during and/or after treatment. In addition, a comparison can be made between the telomerase activity measured during and/or after treatment with a standard level of telomerase activity (historic control). A standard level of telomerase activity is usually determined from a range of telomerase activity levels known to be associated with the particular tumor types and related to or associated with the different clinical stages of the disease. Thus, measurements from historical controls could serve to define a standard level of telomerase activity for a particular cancer and its clinical stage.

In step d) telomerase activity levels in cell samples of cancer patients treated with the anticancer compounds given alone or in combination with chemotherapeutics or radiation are compared with the levels of telomerase activity either before treatment or compared with standard levels of telomerase activity associated with the particular tumor types and related to or associated with the different clinical stages of the disease. A reduction in the level of telomerase activity is indicative for a favourable therapeutic intervention with the growth factor cancer drug. The level of the telomerase activity serves as a basis for adjusting the therapy in order to optimise the treatment schedule with the anticancer drug for the maximum therapeutic benefit to the patient.

The method of the present invention has the following major advantages for cancer therapy:

Measuring telomerase activity provides a basis for monitoring therapeutic efficacy in each individual patient, this basis being available already after a short period of treatment. Furthermore, monitoring the therapeutic efficacy by measuring telomerase activity is applicable to a wide variety of cancers, for example cancer of the lung, breast, head and neck, gastro-intestinal tract, pancrease, bladder, prostate, ovary and others. Thus, a reduction of telomerase activity can be used as a surrogate marker for therapeutically significant antitumor efficacy of growth factor cancer drugs *in vivo.*

In a further aspect, the present invention relates to the use of a telomerase activity detection method for monitoring and evaluating the efficacy of a growth factor cancer drug in therapy.

In a preferred embodiment, the telomerase activity detection method is the telomerase extension method.

Preferably, the telomerase activity detection method is used in the form of a kit, e.g. in the form of the Telomeric Repeat Amplification Protocol.

### Example

Human tumour cells KB (CCL-17) and A431 (CRL-1555) were obtained from ATCC and cultured in DMEM medium supplemented with 10% fetal calf serum, 10 mM HEPES, 50 µmol/l beta-mercaptoethanol and a standard complement of antibiotics.

To establish subcutaneous tumours in nu/nu mice (5-6 week old female animals, supplied by Harlan, The Netherlands), cells were trypsinised, washed and suspended in phosphate-buffered physiological solution + 10% fetal calf serum at 10⁷ cells/ml. 100 µl cell suspension containing 10⁶ cells were then injected subcutaneously into the right flank of the mice (one site per mouse). When tumours were well established and had reached diameters of more than 5-9 mm, mice were randomly distributed between treatment and the vehicle control group (7-10 days after cell injection). Test compounds (1 and 5 mg/ml) were dissolved and administered intragastically by gavage needle. Administration was once daily oral dosing in a volume of 10 ml/kg body weight. Tumour diameters were measured three times weekly with a caliper. Volumes (in mm³) were calculated according to the formula: tumour volume = length * diameter² * π/6.

For measuring telomerase activity, the Oncor®, TRAPeze™ Elisa Telomerase Detection Kit was used. Tumour material surgically removed from mice was cleaned and frozen at -70° C. The preparation of the tissue material extracts and the measurement of the telomerase activity were according to the instructions provided by the supplier.

As growth factor cancer drugs, the compounds designated "Inhibitor 1" and "Inhibitor 2" were administered, which belong to the class of pyrimido-pyrimidines and which are selective for the EGF tyrosine kinase receptor. "Inhibitor 1" is 4-((3-chloro-4-fluoro-phenyl)amino)-6-(1-methyl-4-piperidinyl-amino)-pyrimido(5,4d)pyrimidine; "Inhibitor 2" is 4-((3-chloro-4-fluoro-phenyl)amino)-6-(4-amino-4-methyl-1-piperidinyl)-pyrimido(5,4d)pyrimidine.

After oral administration to *nu/nu* mice xenografted with A431 or KB tumor cells, inhibitors 1 and 2 inhibited tumor growth *in vivo* (Table). Unexpectedly, a massive reduction in telomerase activity was observed in the *in vivo* xenografts after treatment with the growth factor cancer drugs, especially under conditions where a very strong (> 60%) and therapeutically relevant growth inhibition was observed. Consequently, a reduction of telomerase activity can be used as a surrogate marker for therapeutically significant antitumor efficacy of growth factor cancer drugs *in vivo.*

**Table:**

| EGFR inhibition *in vivo* | | | | |
|---|---|---|---|---|
| compound | dose | xenograft | % reduction of telomerase activity | % reduction of tumour growth |
| "Inhibitor 1" | 50 mg/kg | A431 | 100 % | 79 % |
| "Inhibitor 1" | 50 mg/kg | KB | 6 % | 51 % |
| "Inhibitor 2" | 10 mg/kg | A431 | 96 % | 90 % |

## Claims

1. A method for monitoring and evaluating the efficacy of a growth factor cancer drug in therapy, said method comprising the steps of
a) collecting and preparing a sample containing cancer cells from an individual diagnosed for cancer and treated with a growth factor cancer drug,
b) determining the level of telomerase activity in said sample,
c) comparing the level of telomerase activity of said sample with the level determined in a sample in said individual before treatment or with a standard level of telomerase activity, and
d) correlating the level telomerase activity with the therapeutic effect of the growth factor cancer drug.

2. The method of claim 1, wherein in step b) the telomerase level is determined by the extension of a nucleic acid substrate from said sample by telomerase and replication of the extended substrate in a primer extension reaction.

3. The method of claim 2, wherein the primer extension reaction is a polymerase chain reaction.

4. The method of any one of claims 1 to 3, wherein the growth factor cancer drug is an inhibitor of a receptor from the epidermal growth factor receptor family or an inhibitor of the signaling pathway triggered by the activation of a receptor from the epidermal growth factor receptor family.

5. The method of any one of claims 1 to 3, wherein the growth factor cancer drug is an inhibitor of the epidermal growth factor.

6. The method of any one of claims 1 to 3, wherein the growth factor cancer drug is an inhibitor of a receptor from the insulin-like growth factor receptor family or an inhibitor of the signaling pathway triggered by the activation of a receptor from the insulin-like growth factor receptor family.

7. The method of any one of claims 1 to 3, wherein the growth factor cancer drug is an inhibitor of the insulin like growth factor.

8. The method of any one of claims 1 to 3, wherein the growth factor cancer drug is an inhibitor of a receptor from the platelet-derived growth factor receptor family or an inhibitor of the signaling pathway triggered by the activation of a receptor from the platelet-derived growth factor receptor family.

9. The method of any one of claims 1 to 3, wherein the growth factor cancer drug is an inhibitor of the platelet-derived growth factor.

10. The method of any one of claims 1 to 3, wherein the growth factor cancer drug is an inhibitor of a receptor from the neurotrophic factors family or an inhibitor of the signaling pathway triggered by the activation of a receptor from the neurotrophic factors family.

11. The use of a telomerase activity detection method for monitoring and evaluating the efficacy of a growth factor cancer drug in therapy.

12. The use of claim 11, wherein the telomerase activity detection method is the telomerase extension method.

13. The use of claim 11 or 12, wherein the telomerase activity detection method is in the form of a kit.
